(19)

**Europäisches Patentamt**
**European Patent Office**
**Office européen des brevets**

(11) **EP 3 896 095 A1**

(12) **EUROPEAN PATENT APPLICATION**
published in accordance with Art. 153(4) EPC

(43) Date of publication:
**20.10.2021 Bulletin 2021/42**

(51) Int Cl.:
*C08F 20/06* (2006.01)          *C08J 3/12* (2006.01)
*A61F 13/53* (2006.01)          *B01J 20/26* (2006.01)
*B01J 20/28* (2006.01)

(21) Application number: **19895358.0**

(22) Date of filing: **12.12.2019**

(86) International application number:
**PCT/JP2019/048804**

(87) International publication number:
**WO 2020/122207 (18.06.2020 Gazette 2020/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.12.2018  JP 2018232724
12.12.2018  JP 2018232843
12.12.2018  JP 2018232847
12.12.2018  JP 2018232726
12.12.2018  JP 2018232728
12.12.2018  JP 2018232848
12.12.2018  JP 2018232850
12.12.2018  JP 2018232851
12.12.2018  JP 2018232856
12.12.2018  JP 2018232857
30.01.2019  JP 2019014537
22.03.2019  JP 2019055309**

(71) Applicant: **Sumitomo Seika Chemicals Co., Ltd.
Kako-gun, Hyogo 675-0145 (JP)**

(72) Inventor: **OKAZAWA Shiho
Himeji-shi, Hyogo 672-8076 (JP)**

(74) Representative: **Nederlandsch Octrooibureau
P.O. Box 29720
2502 LS The Hague (NL)**

(54) **WATER-ABSORBENT RESIN PARTICLES, ABSORBENT BODY, AND ABSORBENT ARTICLE**

(57) An absorbent article 100 includes an absorber 10, the absorber 10 contains water-absorbent resin particles 10a, in the water-absorbent resin particles 10a, a hardened bulk density is 0.500 g/cm$^3$ or more, and an amount of change in the hardened bulk density at a time of moisture absorption at a temperature of 30°C and a relative humidity of 80% for 1 hour is 0.020 g/cm$^3$ or more.

*Fig.1*

## Description

### Technical Field

The present invention relates to water-absorbent resin particles, an absorber, and an absorbent article.

### Background Art

In the related art, an absorber containing water-absorbent resin particles has been used in an absorbent article for absorbing a liquid (for example, urine) having water as a main component. For example,

Patent Literature 1 below discloses water-absorbent resin particles having a particle diameter that is suitably used for absorbent articles such as diapers. In addition, Patent Literature 2 discloses a method of using a hydrogel-absorbent polymer having specific saline flow inducibility, performance under pressure, and the like as an effective absorbent member for storing a body fluid such as urine.

### Citation List

### Patent Literature

[Patent Literature 1] Japanese Unexamined Patent Publication No. H6-345819
[Patent Literature 2] Japanese Unexamined Patent Publication No. H9-510889

## Summary of Invention

### Technical Problem

In a case where a liquid provided to an absorbent article does not sufficiently permeate the absorbent article, there may occur a problem that the excess liquid leaks to the outside of the absorbent article, for example, the excess liquid flows on the surface thereof. Therefore, it is required that the liquid permeates the absorbent article at a better permeation rate.

An object of an aspect of the present invention is to provide water-absorbent resin particles capable of obtaining an absorbent article having a better permeation rate. In addition, an object of another aspect of the present invention is to provide an absorber and an absorbent article using the water-absorbent resin particles.

### Solution to Problem

The present inventor has paid attention to a hardened bulk density of water-absorbent resin particles on the basis of the fact that a mechanical load or impact is applied to the water-absorbent resin particles during the production, the use, or the like of the absorbent article, and has found that adjusting the hardened bulk density and its amount of change during moisture absorption is effective in obtaining an absorbent article having a better permeation rate.

An aspect of the present invention provides water-absorbent resin particles in which a hardened bulk density is 0.500 $g/cm^3$ or more, and an amount of change in the hardened bulk density at a time of moisture absorption at a temperature of 30°C and a relative humidity of 80% for 1 hour is 0.020 $g/cm^3$ or more.

According to the above-mentioned water-absorbent resin particles, it is possible to obtain an absorbent article having a better permeation rate.

Another aspect of the present invention provides an absorber containing the above-mentioned water-absorbent resin particles.

Another aspect of the present invention provides a water-absorbent article including the above-mentioned absorber.

### Advantageous Effects of Invention

According to an aspect of the present invention, it is possible to provide water-absorbent resin particles capable of obtaining an absorbent article having a better permeation rate. In addition, according to another aspect of the present invention, it is possible to provide an absorber and an absorbent article using the water-absorbent resin particles. According to another aspect of the present invention, it is possible to provide use of a resin particle, an absorber, and an absorbent article to the absorption of a liquid.

## Brief Description of Drawings

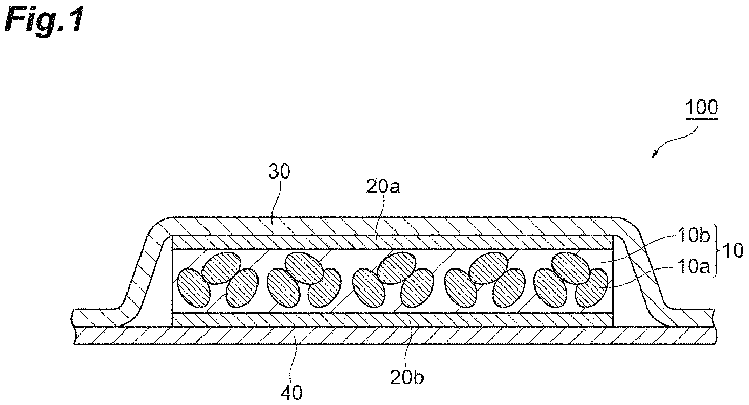

Fig. 1 is a cross-sectional view showing an example of an absorbent article.

## Description of Embodiments

Hereinafter, embodiments of the present invention will be described in detail. However, the present invention is not limited to the following embodiments, and can be variously modified and implemented within the scope of the gist thereof.

In the present specification, "acrylic" and "methacryl" are collectively referred to as "(meth)acrylic". Similarly, "acrylate" and "methacrylate" are also referred to as "(meth)acrylate". In a numerical value range described in stages in the present specification, an upper limit value or a lower limit value of the numerical value range of a stage can be optionally combined with the upper limit value or the lower limit value of the numerical value range of another stage. In a numerical value range described in the present specification, the upper limit value or the lower limit value of the numerical value range may be replaced with the value shown in the examples. "Water-soluble" means that it exhibits a solubility in water of 5% by mass or more at 25°C. Materials exemplified in the present specification may be used alone, or may be used in combination of two or more. The content of each component in the composition means the total amount of a plurality of substances present in the composition in a case where the plurality of substances corresponding to each component are present in the composition, unless otherwise specified. "Physiological saline" refers to 0.9% by mass sodium chloride aqueous solution.

In the present embodiment, the hardened bulk density of the water-absorbent resin particles is 0.500 $g/cm^3$ ($g/cm^3$ = g/mL) or more, and an amount of change in the hardened bulk density of the water-absorbent resin particles when the water-absorbent resin particles are allowed to absorb moisture at the temperature of 30°C and the relative humidity of 80% for 1 hour is 0.020 $g/cm^3$ or more. According to such water-absorbent resin particles, it is possible to obtain an absorbent article having a better permeation rate. The cause of obtaining such an effect is not clear, but the present inventor makes presumptions as follows. However, the cause is not limited to the following contents.

Specifically, in a case where the hardened bulk density is 0.500 $g/cm^3$ or more and an amount of change in the hardened bulk density is 0.020 $g/cm^3$ or more, since the water-absorbent resin particles are easily compressed after moisture absorption in a space of the same volume, and since the viscosity of the surface of the compressed water-absorbent resin particles and the like is appropriately changed (improved), the water-absorbent resin particles are easily fixed to each other or to other components (for example, a fibrous substance). Due to such fixation, the mutual contact area of the water-absorbent resin particles or the contact area between the water-absorbent resin particles and other components (for example, a fibrous substance) is large, and therefore, the liquid is easily quickly absorbed in the water-absorbent resin particles (for example, the liquid easily moves from the fibrous substance to the water-absorbent resin particles). Therefore, it is presumed that a better permeation rate is obtained.

The hardened bulk density of the water-absorbent resin particles is the mass of the water-absorbent resin particles with respect to the volume when a container is tapped after the water-absorbent resin particles are filled in the container. The hardened bulk density can be measured by the method described in examples to be described later.

In the present embodiment, from a viewpoint of obtaining a better permeation rate in the absorbent article, the hardened bulk density of the water-absorbent resin particles before moisture absorption is 0.500 $g/cm^3$ or more. The hardened bulk density before moisture absorption is preferably 0.600 $g/cm^3$ or more, 0.700 $g/cm^3$ or more, 0.750 $g/cm^3$ or more, 0.800 $g/cm^3$ or more, more than 0.800 $g/cm^3$, 0.810 $g/cm^3$ or more, more than 0.810 $g/cm^3$, 0.820 $g/cm^3$ or more, 0.830 $g/cm^3$ or more, 0.840 $g/cm^3$ or more, or 0.845 $g/cm^3$ or more, from a viewpoint of easily obtaining a better permeation rate in an absorbent article. The hardened bulk density before moisture absorption may be 0.850 $g/cm^3$ or more, 0.860 $g/cm^3$ or more, or 0.870 $g/cm^3$ or more. The hardened bulk density before moisture absorption is preferably 1.500 $g/cm^3$ or less, 1.400 $g/cm^3$ or less, 1.200 $g/cm^3$ or less, 1.000 $g/cm^3$ or less, 0.950 $g/cm^3$ or less, 0.940 $g/cm^3$ or less, 0.920 $g/cm^3$ or less, 0.900 $g/cm^3$ or less, 0.890 $g/cm^3$ or less, 0.880 $g/cm^3$ or less, 0.870 $g/cm^3$ or less, 0.860 $g/cm^3$ or less, or 0.850 $g/cm^3$ or less, from a viewpoint of easily obtaining a better permeation rate in an absorbent article. From these viewpoints, the hardened bulk density before moisture absorption is preferably 0.500 to 0.950 $g/cm^3$, more preferably 0.600 to 0.950 $g/cm^3$, and further more preferably 0.830 to 0.950 $g/cm^3$. As the hardened bulk density before moisture absorption, a hardened bulk density at room temperature (25°C $\pm$ 2°C) can be used.

In the present embodiment, from a viewpoint of obtaining a better permeation rate in the absorbent article, an amount of change in the hardened bulk density of the water-absorbent resin particles when the water-absorbent resin particles absorb moisture at the temperature of 30°C and the relative humidity of 80% for 1 hour (amount of change in the hardened bulk density after moisture absorption) is 0.020 $g/cm^3$ or more. In a case where the water-absorbent resin particles absorb moisture, the hardened bulk density tends to decrease, and the hardened bulk density after moisture absorption tends to be smaller than that before moisture absorption. By using conditions of the temperature of 30°C and the relative humidity of 80%, accelerated evaluation assuming a high temperature and a high humidity area is possible, and it is easy to select water-absorbent resin particles that provides a better permeation rate in an absorbent article. As the amount of change in the hardened bulk density after moisture absorption, an amount of change at room temperature (25 ± 2°C) can be used.

The amount of change in the hardened bulk density after moisture absorption is preferably 0.025 g/cm$^3$ or more, 0.030 g/cm$^3$ or more, 0.031 g/cm$^3$ or more, 0.032 g/cm$^3$ or more, 0.033 g/cm$^3$ or more, 0.034 g/cm$^3$ or more, more than 0.034 g/cm$^3$, or 0.035 g/cm$^3$ or more, from a viewpoint of easily obtaining a better permeation rate in the absorbent article. The amount of change in the hardened bulk density after moisture absorption is preferably 0.060 g/cm$^3$ or less, 0.055 g/cm$^3$ or less, 0.050 g/cm$^3$ or less, 0.049 g/cm$^3$ or less, less than 0.049 g/cm$^3$, 0.048 g/cm$^3$ or less, 0.045 g/cm$^3$ or less, 0.043 g/cm$^3$ or less, 0.041 g/cm$^3$ or less, 0.040 g/cm$^3$ or less, or 0.038 g/cm$^3$ or less, from a viewpoint of not significantly changing a sense of wearing during use of the absorbent article. From these viewpoints, the amount of change in the hardened bulk density after moisture absorption is preferably 0.020 to 0.060 g/cm$^3$.

The hardened bulk density of the water-absorbent resin particles after the water-absorbent resin particles absorb moisture at the temperature of 30°C and the relative humidity of 80% for 1 hour (hardened bulk density after moisture absorption) is preferably 0.570 g/cm$^3$ or more, 0.670 g/cm$^3$ or more, 0.720 g/cm$^3$ or more, 0.750 g/cm$^3$ or more, 0.760 g/cm$^3$ or more, 0.770 g/cm$^3$ or more, 0.780 g/cm$^3$ or more, more than 0.780 g/cm$^3$, 0.790 g/cm$^3$ or more, 0.800 g/cm$^3$ or more, or 0.810 g/cm$^3$ or more, from a viewpoint of easily obtaining a better permeation rate in the absorbent article. The hardened bulk density after moisture absorption is 0.910 g/cm$^3$ or less, 0.890 g/cm$^3$ or less, 0.870 g/cm$^3$ or less, 0.860 g/cm$^3$ or less, 0.850 g/cm$^3$ or less, 0.840 g/cm$^3$ or less, 0.830 g/cm$^3$ or less, or 0.820 g/cm$^3$ or less, from a viewpoint of easily obtaining a better permeation rate in the absorbent article. From these viewpoints, the hardened bulk density after moisture absorption is preferably 0.570 to 0.910 g/cm$^3$. As the hardened bulk density after moisture absorption, the hardened bulk density at room temperature (25°C ± 2°C) can be used.

The water-absorbent resin particles of the present embodiment may be any water-absorbent resin particles as long as the water-absorbent resin particles can retain water, and the liquid to be absorbed can contain water. The water-absorbent resin particles of the present embodiment are better in absorbency of a body fluid such as urine, sweat, blood (for example, menstrual blood). The water-absorbent resin particles of the present embodiment can be used as a constituent component of the absorber of the present embodiment.

The water content of the water-absorbent resin particles of the present embodiment before moisture absorption (the water content of the water-absorbent resin particles which provide the hardened bulk density of 0.500 g/cm$^3$ or more), and the water content of the water-absorbent resin particles of the present embodiment after the water-absorbent resin particles are caused to absorb moisture at the temperature of 30°C and the relative humidity of 80% for 1 hour (the water content after moisture absorption) are preferably in the following range. The water content can be measured by the method described in examples to be described later.

The water content before moisture absorption is preferably 1% by mass or more, 3% by mass or more, 5% by mass or more, 6% by mass or more, 7% by mass or more, or 8% by mass or more, from a viewpoint of easily obtaining an appropriate initial water absorption rate of the water-absorbent resin particles. The water content before moisture absorption is preferably 12% by mass or less, 11% by mass or less, 10% by mass or less, or 9% by mass or less, from a viewpoint of easily obtaining appropriate flowability of the water-absorbent resin particles. From these viewpoints, the water content before moisture absorption is preferably 1 to 12% by mass. The water content before moisture absorption may be 0% by mass or exceed 0% by mass.

The water content after moisture absorption is preferably 5% by mass or more, 8% by mass or more, 10% by mass or more, 11% by mass or more, 12% by mass or more, 13% by mass or more, or 14% by mass or more, from a viewpoint of easily obtaining a better permeation rate in the absorbent article. The water content after moisture absorption is preferably 20% by mass or less, 18% by mass or less, 16% by mass or less, or 15% by mass or less, from a viewpoint of easily obtaining a better permeation rate in the absorbent article. From these viewpoints, the water content after moisture absorption is preferably 5% to 20% by mass.

The water retention amount of physiological saline of the water-absorbent resin particles of the present embodiment is preferably in the following range. The water retention amount is preferably 10 g/g or more, 15 g/g or more, 20 g/g or more, 25 g/g or more, 30 g/g or more, 35 g/g or more, or 40 g/g or more, from a viewpoint of easily obtaining a better permeation rate in the absorbent article. The water retention amount is preferably 80 g/g or less, 75 g/g or less, 70 g/g or less, 65 g/g or less, 60 g/g or less, 55 g/g or less, 50 g/g or less, 45 g/g or less, 44 g/g or less, 43 g/g or less, 42 g/g or less, or 41 g/g or less, from a viewpoint of easily obtaining better permeation rate in the absorbent article. From these viewpoints, the water retention amount is preferably 10 to 80 g/g, more preferably 30 to 55 g/g, further more preferably 35 to 50 g/g, and particularly preferably 40 to 45 g/g. As the water retention amount, the water retention amount at room temperature (25°C ± 2°C) can be used. The water retention amount can be measured by the method described in examples to be described later.

Examples of the shape of the water-absorbent resin particles of the present embodiment include substantially spherical, crushed, and granular shapes. The medium particle diameter of the water-absorbent resin particles (the water-absorbent resin particles before moisture absorption) of the present embodiment is preferably in the following range. The medium particle diameter is preferably 250 $\mu$m or more, 280 $\mu$m or more, 300 $\mu$m or more, 310 $\mu$m or more, or 320 $\mu$m or more from a viewpoint of avoiding gel blocking and easily maintaining a good permeation rate of the absorbent article. The medium particle diameter is preferably 600 $\mu$m or less, 550 $\mu$m or less, 500 $\mu$m or less, 450 $\mu$m or less, 400 $\mu$m or less, 380 $\mu$m or less, 360 $\mu$m or less, 350 $\mu$m or less, 340 $\mu$m or less, or 330 $\mu$m or less from a viewpoint of easily keeping the touch feeling of the absorbent article soft. From these viewpoints, the medium particle diameter is preferably 250 to 600 $\mu$m. The water-absorbent resin particles of the present embodiment may have a desired particle size distribution at the time of being obtained by a production method to be described later, but the particle size distribution may be adjusted by performing an operation such as particle size adjustment using classification with a sieve.

The water-absorbent resin particles of the present embodiment can contain a crosslinking polymer (a crosslinking polymer having a structural unit derived from an ethylenically unsaturated monomer) obtained by polymerizing a monomer containing an ethylenically unsaturated monomer, as polymer particles, for example. That is, the water-absorbent resin particles of the present embodiment can have a structural unit derived from an ethylenically unsaturated monomer, and can contain polymer particles including a crosslinking polymer having a structural unit derived from an ethylenically unsaturated monomer. As the ethylenically unsaturated monomer, a water-soluble ethylenically unsaturated monomer can be used. Examples of the polymerization method include a reverse phase suspension polymerization method, an aqueous solution polymerization method, a bulk polymerization method, and a precipitation polymerization method. Among these, the reverse phase suspension polymerization method or the aqueous solution polymerization method is preferable from a viewpoint of ensuring good hardened bulk density and its amount of change, water-absorbent characteristics (water retention amount and the like) and the like of the obtained water-absorbent resin particles and facilitating control of the polymerization reaction. In the following, as a method for polymerizing an ethylenically unsaturated monomer, a reverse phase suspension polymerization method will be described as an example.

The ethylenically unsaturated monomer is preferably water-soluble, and examples thereof include (meth)acrylic acid and a salt thereof, 2-(meth)acrylamide-2-methylpropanesulfonic acid and a salt thereof, (meth)acrylamide, N, N-dimethyl (meth)acrylamide, 2-hydroxyethyl (meth)acrylate, N-methylol (meth)acrylamide, polyethylene glycol mono(meth)acrylate, N, N-diethylaminoethyl (meth)acrylate, N, N-diethylaminopropyl (meth)acrylate, and diethylaminopropyl (meth)acrylamide. In a case where the ethylenically unsaturated monomer has an amino group, the amino group may be quaternized. The ethylenically unsaturated monomer may be used alone, or may be used in combination of two or more. Functional groups such as a carboxyl group and an amino group of the above-mentioned monomers can function as functional groups capable of crosslinking in a surface crosslinking step to be described later.

Among these, from a viewpoint of industrial availability, the ethylenically unsaturated monomer preferably contains at least one compound selected from the group consisting of (meth)acrylic acid and a salt thereof, acrylamide, methacrylamide, and N, N-dimethylacrylamide, and more preferably contains at least one compound selected from the group consisting of (meth)acrylic acid and a salt thereof, and acrylamide. From a viewpoint of further enhancing the water-absorbent characteristics (water retention amount, and the like), the ethylenically unsaturated monomer further more preferably contains at least one compound selected from the group consisting of (meth)acrylic acid and a salt thereof. That is, the water-absorbent resin particles preferably have a structural unit derived from at least one selected from the group consisting of (meth)acrylic acid and a salt thereof.

As the monomer for obtaining the water-absorbent resin particles, a monomer other than the above-mentioned ethylenically unsaturated monomer may be used. Such a monomer can be used by being mixed with an aqueous solution containing the above-mentioned ethylenically unsaturated monomer, for example. The use amount of the ethylenically unsaturated monomer is preferably 70 to 100 mol% with respect to a total amount of the monomer (the total amount of the monomer for obtaining the water-absorbent resin particles. For example, a total amount of the monomers that provide a structural unit of the crosslinking polymer. The same applies hereinafter). Among these, the ratio of (meth)acrylic acid and a salt thereof is more preferably 70 to 100 mol% with respect to the total amount of the monomers. "Ratio of (meth)acrylic acid and a salt thereof" means the ratio of the total amount of (meth)acrylic acid and a salt thereof.

According to the present embodiment, as an example of the water-absorbent resin particles, it is possible to provide the water-absorbent resin particles containing a crosslinking polymer having a structural unit derived from an ethylenically unsaturated monomer, in which the ethylenically unsaturated monomer contains at least one compound selected from the group consisting of (meth)acrylic acid and a salt thereof, the ratio of (meth)acrylic acid and a salt thereof is 70 to 100 mol% with respect to the total amount of the monomer for obtaining the water-absorbent resin particles (for example, the total amount of the monomer that provides a structural unit of the crosslinking polymer), the hardened bulk density is 0.500 g/cm$^3$ or more, and the amount of change in the hardened bulk density at the time of moisture absorption at the temperature of 30°C and the relative humidity of 80% for 1 hour is 0.020 g/cm$^3$ or more. In addition, according to the present embodiment, as an example of the water-absorbent resin particles, it is possible to provide the water-absorbent resin particles containing polymer particles containing a crosslinking polymer having a structural unit derived from an ethylenically unsaturated monomer, and inorganic particles disposed on a surface of the polymer particles, in which the ethylenically unsaturated monomer includes at least one compound selected from the group consisting of (meth)acrylic acid and a salt thereof, the ratio of (meth)acrylic acid and a salt thereof is 70 to 100 mol% with respect to the total amount of the monomer for obtaining the water-absorbent resin particles (for example, total amount of the monomer that provides a structural unit of the crosslinking polymer), the hardened bulk density is 0.500 g/cm$^3$ or more, and the amount of change in the hardened bulk density at the time of moisture absorption at the temperature of 30°C and the relative humidity of 80% for 1 hour is 0.020 g/cm$^3$ or more.

The ethylenically unsaturated monomer is usually preferably used as an aqueous solution. The concentration of the ethylenically unsaturated monomer in the aqueous solution containing the ethylenically unsaturated monomer (hereinafter, simply referred to as "monomer aqueous solution") is preferably 20% by mass or more and a saturated concentration or less, more preferably 25 to 70% by mass, and further more preferably 30 to 55% by mass. Examples of the water used in the aqueous solution include tap water, distilled water, and ion-exchanged water.

In a case where the ethylenically unsaturated monomer has an acid group, the monomer aqueous solution may be used by neutralizing the acid group with an alkaline neutralizing agent. The degree of neutralization of the ethylenically unsaturated monomer by the alkaline neutralizing agent is preferably 10 to 100 mol%, more preferably 50 to 90 mol%, and further more preferably 60 to 80 mol% of the acid group in the ethylenically unsaturated monomer, from a viewpoint of increasing an osmotic pressure of the obtained water-absorbent resin particles, and further increasing the water-absorbent characteristics (water retention amount and the like). Examples of the alkaline neutralizing agent include alkali metal salts such as sodium hydroxide, sodium carbonate, sodium hydrogen carbonate, potassium hydroxide, and potassium carbonate; and ammonia. The alkaline neutralizing agent may be used alone, or may be used in combination of two or more. The alkaline neutralizing agent may be used in the form of an aqueous solution to simplify the neutralization operation. Neutralization of the acid group of the ethylenically unsaturated monomer can be performed by adding an aqueous solution of sodium hydroxide, potassium hydroxide, or the like dropwise in the above-mentioned monomer aqueous solution and mixing therewith.

In a reverse phase suspension polymerization method, a monomer aqueous solution is dispersed in a hydrocarbon dispersion medium in the presence of a surfactant, and polymerization of the ethylenically unsaturated monomer can be performed using a radical polymerization initiator or the like. As the radical polymerization initiator, a water-soluble radical polymerization initiator can be used.

Examples of the surfactant include a nonionic surfactant, and an anionic surfactant. Examples of the nonionic surfactant include sorbitan fatty acid ester, (poly)glycerin fatty acid ester ("(poly)" means both of a case where there is a prefix of "poly" and a case where there is no prefix thereof. The same applies hereinafter), sucrose fatty acid ester, polyoxyethylene sorbitan fatty acid ester, polyoxyethylene glycerin fatty acid ester, sorbitol fatty acid ester, polyoxyethylene sorbitol fatty acid ester, polyoxyethylene alkyl ether, polyoxyethylene alkyl phenyl ether, polyoxyethylene castor oil, polyoxyethylene hydrogenated castor oil, alkylallyl formaldehyde condensed polyoxyethylene ether, polyoxyethylene polyoxypropylene block copolymer, polyoxyethylene polyoxypropyl alkyl ether, and polyethylene glycol fatty acid ester. Examples of the anionic surfactant include fatty acid salt, alkylbenzene sulfonate, alkylmethyl taurate, polyoxyethylene alkylphenyl ether sulfate, polyoxyethylene alkyl ether sulfonate, phosphate ester of polyoxyethylene alkyl ether, and phosphate ester of polyoxyethylene alkylallyl ether. The surfactant may be used alone, or may be used in combination of two or more.

From a viewpoint of a good state of the W/O type reverse phase suspension, easily obtaining water-absorbent resin particles having a suitable particle diameter, and industrial availability, the surfactant preferably contains at least one compound selected from the group consisting of a sorbitan fatty acid ester, a polyglycerin fatty acid ester, and a sucrose fatty acid ester. From a viewpoint of easily obtaining an appropriate particle size distribution of the water-absorbent resin particles, and from a viewpoint of easily improving water-absorbent characteristics (water retention amount and the like) of the water-absorbent resin particles and performance of the absorbent article using the same, the surfactant preferably contains sucrose fatty acid ester, and more preferably contains sucrose stearic acid ester.

The use amount of the surfactant is preferably 0.05 to 10 parts by mass, more preferably 0.08 to 5 parts by mass, and further more preferably 0.1 to 3 parts by mass with respect to 100 parts by mass of the monomer aqueous solution from a viewpoint of obtaining a sufficient effect on the use amount and economic efficiency.

In the reverse phase suspension polymerization, a polymeric dispersant may be used in combination with the above-mentioned surfactant. Examples of the polymeric dispersant include maleic anhydride-modified polyethylene, maleic anhydride-modified polypropylene, maleic anhydride-modified ethylene/propylene copolymer, maleic anhydride-modified EPDM (ethylene propylene diene terpolymer), maleic anhydride-modified polybutadiene, maleic anhydride/ethylene copolymer, maleic anhydride/propylene copolymer, maleic anhydride/ethylene/propylene copolymer, maleic anhydride/butadiene copolymer, polyethylene, polypropylene, ethylene/propylene copolymer, oxidized polyethylene, oxidized polypropylene, oxidized ethylene/propylene copolymer, ethylene/acrylic acid copolymer, ethyl cellulose, and ethyl hydroxyethyl cellulose. The polymeric dispersant may be used alone or may be used in combination of two or more. From a viewpoint of better dispersion stability of the monomer, the polymeric dispersant is preferably at least one selected from the group consisting of maleic anhydride-modified polyethylene, maleic anhydride-modified polypropylene, maleic anhydride-modified ethylene/propylene copolymer, maleic anhydride/ethylene copolymer, maleic anhydride/propylene copolymer, maleic anhydride/ethylene/propylene copolymer, polyethylene, polypropylene, ethylene/propylene copolymer, oxidized polyethylene, oxidized polypropylene, and oxidized ethylene/propylene copolymer.

The use amount of the polymeric dispersant is preferably 0.05 to 10 parts by mass, more preferably 0.08 to 5 parts by mass, and further more preferably 0.1 to 3 parts by mass with respect to 100 parts by mass of the monomer aqueous solution, from a viewpoint of obtaining a sufficient effect on the use amount and economic efficiency.

The hydrocarbon dispersion medium may contain at least one compound selected from the group consisting of chain aliphatic hydrocarbons having 6 to 8 carbon atoms and alicyclic hydrocarbons having 6 to 8 carbon atoms. Examples of the hydrocarbon dispersion medium include chain aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone, or may be used in combination of two or more.

The hydrocarbon dispersion medium may contain at least one selected from the group consisting of n-heptane and cyclohexane from a viewpoint of industrial availability and stable quality. In addition, from the same viewpoint, as the mixture of the above-mentioned hydrocarbon dispersion medium, for example, commercially available Exxsol Heptane (manufactured by ExxonMobil: containing 75% to 85% of n-heptane and isomeric hydrocarbons) may be used.

The use amount of the hydrocarbon dispersion medium is preferably 30 to 1000 parts by mass, more preferably 40 to 500 parts by mass, and further more preferably 50 to 400 parts by mass with respect to 100 parts by mass of the monomer aqueous solution, from a viewpoint of appropriately removing the heat of polymerization and easily controlling the polymerization temperature. In a case where the use amount of the hydrocarbon dispersion medium is 30 parts by mass or more, the polymerization temperature tends to be easily controlled. In a case where the use amount of the hydrocarbon dispersion medium is 1000 parts by mass or less, the productivity of polymerization tends to be improved, which is economical.

The radical polymerization initiator is preferably water-soluble, and examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumylperoxide, t-butylperoxyacetate, t-butylperoxyisobutyrate, t-butylperoxypivalate, and hydrogen peroxide; azo compounds such as 2,2'-azobis(2-amidinopropane) dihydrochloride, 2,2'-azobis [2-(N-phenylamidino) propane] dihydrochloride, 2,2'-azobis [2-(N-allylamidino) propane] dihydrochloride, 2,2'-azobis [2-(2-imidazoline-2-yl) propane] dihydrochloride, 2,2'-azobis {2-[1-(2-hydroxyethyl) -2-imidazoline-2-yl] propane} dihydrochloride, 2,2'-azobis {2-methyl-N-[1,1-bis (hydroxymethyl)-2-hydroxyethyl] propionamide}, 2,2'-azobis [2-methyl-N-(2-hydroxyethyl)-propionamide], and 4,4'-azobis (4-cyanovaleric acid). The radical polymerization initiator may be used alone, or may be used in combination of two or more. The radical polymerization initiator is preferably at least one selected from the group consisting of potassium persulfate, ammonium persulfate, sodium persulfate, 2,2'-azobis (2-amidinopropane) dihydrochloride, 2,2'-azobis [2- (2-imidazoline-2-yl) propane] dihydrochloride, and 2,2'-azobis {2-[1-(2-hydroxyethyl)-2-imidazoline-2-yl] propane} dihydrochloride.

The use amount of the radical polymerization initiator may be 0.05 to 10 mmol with respect to 1 mol of the ethylenically unsaturated monomer. In a case where the use amount of the radical polymerization initiator is 0.05 mmol or more, the polymerization reaction does not require a long time and is efficient. In a case where the use amount of the radical polymerization initiator is 10 mmol or less, the occurrence of a rapid polymerization reaction is easily inhibited.

The above-mentioned radical polymerization initiator can also be used as a redox polymerization initiator in combination with a reducing agent such as sodium sulfite, sodium hydrogen sulfite, ferrous sulfate, and L-ascorbic acid.

At the time of the polymerization reaction, the monomer aqueous solution used for the polymerization may contain a chain transfer agent. Examples of the chain transfer agent include hypophosphites, thiols, thiolic acids, secondary alcohols, and amines.

The monomer aqueous solution used for the polymerization may contain a thickener in order to control the particle diameter of the water-absorbent resin particles. Examples of the thickener include hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, and polyethylene oxide. In a case where the stirring speed at the time of polymerization is the same, the higher the viscosity of the monomer aqueous solution, the larger the medium particle diameter of the obtained particles tends to be.

Crosslinking by self-crosslinking may occur during polymerization, but crosslinking may be performed by using an internal crosslinking agent. In a case where an internal crosslinking agent is used, the hardened bulk density and its amount of change, water-absorbent characteristics (water retention amount and the like), and the like of the water-absorbent resin particles are easily controlled. The internal crosslinking agent is usually added to a reaction solution during the polymerization reaction. Examples of the internal crosslinking agent include di or tri (meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the above-mentioned polyols with unsaturated acids (such as maleic acid and fumaric acid); bis (meth)acrylamides such as N, N'-methylene bis (meth)acrylamide; di or tri (meth)acrylic acid esters obtained by reacting polyepoxide with (meth)acrylic acid; di (meth)acrylic acid carbamil esters obtained by reacting polyisocyanate (such as tolylene diisocyanate and hexamethylene diisocyanate) with hydroxyethyl (meth)acrylate; compounds having two or more polymerizable unsaturated groups such as allylated starch, allylated cellulose, diallyl phthalate, N, N', N''-triallyl isocyanurate, and divinylbenzene; polyglycidyl compounds such as (poly)ethylene glycol diglycidyl ether, (poly) propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromhydrin, and $\alpha$-methylepichlorohydrin; and compounds having two or more reactive functional groups such as isocyanate compounds (2,4-tolylene diisocyanate and hexamethylene diisocyanate). The internal crosslinking agent may be used alone, or may be used in combination of two or more. The internal crosslinking agent is preferably a polyglycidyl compound, more preferably a diglycidyl ether compound, and further more preferably at least one selected from the group consisting of (poly)ethylene glycol diglycidyl ether, (poly)propylene glycol diglycidyl ether, and (poly)glycerin diglycidyl ether.

The use amount of the internal crosslinking agent is preferably 30 mmol or less, more preferably 0.01 to 10 mmol, further more preferably 0.012 to 5 mmol, particularly preferably 0.015 to 1 mmol, extremely preferably 0.02 to 0.1 mmol, and extraordinarily preferably 0.025 to 0.06 mmol per 1 mol of the ethylenically unsaturated monomer, from a viewpoint of easily adjusting the hardened bulk density and its amount of change, and from a viewpoint of suppressing water-soluble property by appropriately crosslinking the obtained polymer to easily obtain the sufficient water absorption amount.

It is possible to perform heating while stirring in a state of mixing an ethylenically unsaturated monomer, a radical polymerization initiator, a surfactant, a polymeric dispersant, a hydrocarbon dispersion medium, or the like (if necessary, additionally an internal crosslinking agent), and to perform reverse phase suspension polymerization in a water-in-oil system.

When performing the reverse phase suspension polymerization, a monomer aqueous solution containing an ethylenically unsaturated monomer is dispersed in a hydrocarbon dispersion medium in the presence of a surfactant (if necessary, additionally a polymeric dispersant). At this time, before the start of the polymerization reaction, the timing of adding the surfactant, the polymeric dispersant, or the like may be either before or after the addition of the monomer aqueous solution.

Among these, from a viewpoint of easily reducing the amount of the hydrocarbon dispersion medium remaining in the obtained water-absorbent resin, it is preferable to perform polymerization after dispersing the monomer aqueous solution in the hydrocarbon dispersion medium in which the polymeric dispersant is dispersed and then further dispersing the surfactant.

Reverse phase suspension polymerization can be performed in one stage, or in multiple stages of two or more stages. Reverse phase suspension polymerization is preferably performed in two to three stages from a viewpoint of increasing productivity.

In a case where reverse phase suspension polymerization is performed in multiple stages of two or more stages, a first stage reverse phase suspension polymerization is performed, an ethylenically unsaturated monomer is added to the reaction mixture obtained in the first polymerization reaction and mixed therewith, and second and subsequent stages of reverse phase suspension polymerization may be performed in the same method as the first stage. In the reverse phase suspension polymerization in each stage of the second and subsequent stages, in addition to the ethylenically unsaturated monomer, the above-mentioned radical polymerization initiator and/or internal crosslinking agent is preferably added in a range of a molar ratio of each component with respect to the above-mentioned ethylenically unsaturated monomer, based on an amount of the ethylenically unsaturated monomer added at the time of the second and subsequent stages of reverse phase suspension polymerization, to perform reverse phase suspension polymerization. In the reverse phase suspension polymerization in each stage of second and subsequent stages, an internal crosslinking agent may be used if necessary. In a case of using the internal crosslinking agent, the internal crosslinking agent is preferably added within a range of the molar ratio of each component with respect to the above-mentioned ethylenically unsaturated monomer based on the amount of the ethylenically unsaturated monomer provided in each stage, to perform reverse phase suspension polymerization.

The temperature of the polymerization reaction varies depending on the used radical polymerization initiator, and the temperature is preferably 20°C to 150°C, and more preferably 40°C to 120°C, from a viewpoint of rapidly proceeding the polymerization and shortening the polymerization time to enhance economic efficiency, and easily removing polymerization heat and smoothly performing reaction. The reaction time is usually 0.5 to 4 hours. The completion of the polymerization reaction can be confirmed by stopping the temperature rise in the reaction system. Thus, the polymer of the ethylenically unsaturated monomer is usually obtained in a state of a hydrogel.

After the polymerization, a post-polymerization crosslinking agent may be added to the obtained hydrogel-like polymer and heated to perform crosslinking. By performing post-polymerization crosslinking, a degree of crosslinking of the hydrogel-like polymer can be increased, and the water-absorbent characteristics (water retention amount, and the like) can be further improved.

Examples of the post-polymerization crosslinking agent include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups such as (poly)ethylene glycol diglycidyl ether, (poly)propylene glycol diglycidyl ether, and (poly)glycerin diglycidyl ether; haloepoxy compounds such as epichlorohydrin, epibromhydrin, and $\alpha$-methylepichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis [N, N-di ($\beta$-hydroxyethyl)] adipamide. Among these, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. The crosslinking agent may be used alone, or may be used in combination of two or more.

The amount of the post-polymerization crosslinking agent is preferably 30 mmol or less, more preferably 10 mmol or less, further more preferably 0.01 to 5 mmol, particularly preferably 0.012 to 1 mmol, extremely preferably 0.015 to 0.1 mmol, and extraordinarily preferably 0.02 to 0.05 mmol per 1 mol of the ethylenically unsaturated monomer, from a viewpoint of easily obtaining suitable hardened bulk density and its amount of change, water-absorbent characteristics (water retention amount and the like), and the like by appropriately crosslinking the obtained hydrogel-like polymer.

The timing of adding the post-polymerization crosslinking agent may be after the polymerization of the ethylenically unsaturated monomer used for the polymerization, and in the case of multiple-stage polymerization, it is preferable to add after the multiple-stage polymerization. Considering fluctuation in water due to heat generation at the time of polymerization and after polymerization, retention due to process delay, opening of the system at the time of addition of the crosslinking agent, addition of water due to the addition of the crosslinking agent, or the like, the post-polymerization crosslinking agent is preferably added in a region of [water content (immediately after polymerization) ± 3% by mass] from a viewpoint of water content (to be described later).

Subsequently, the polymer particles (for example, polymer particles having a structural unit derived from an ethylenically unsaturated monomer) are obtained by drying in order to remove water from the obtained hydrogel-like polymer. Examples of a drying method include (a) a method of removing water by performing azeotropic distillation by heating from outside in a state where a hydrogel-like polymer is dispersed in a hydrocarbon dispersion medium, and refluxing the hydrocarbon dispersion medium, (b) a method of taking out a hydrogel-like polymer by decantation and drying under reduced pressure, and (c) a method of filtering the hydrogel-like polymer with a filter and drying under reduced pressure. Among these, it is preferable to use the method (a) due to the simplicity in the production process.

It is possible to adjust the particle diameter of water-absorbent resin particles by adjusting a rotation speed of a stirrer during the polymerization reaction, or by adding a flocculant into the system after the polymerization reaction or in the initial stage of drying. By adding a flocculant, it is possible to increase the particle diameter of the obtained water-absorbent resin particles. As the flocculant, an inorganic flocculant can be used. Examples of the inorganic flocculant (for example, powdered inorganic flocculant) include silica, zeolite, bentonite, aluminum oxide, talc, titanium dioxide, kaolin, clay, and hydrotalcite. From a viewpoint of better flocculation effect, the flocculant is preferably at least one selected from the group consisting of silica, aluminum oxide, talc, and kaolin.

In the reverse phase suspension polymerization, a method of adding the flocculant is preferably a method of preliminarily dispersing a flocculant in a hydrocarbon dispersion medium or water of the same type as that used in the polymerization, and then mixing into a hydrocarbon dispersion medium containing a hydrogel-like polymer under stirring.

The addition amount of the flocculant is preferably 0.001 to 1 part by mass, more preferably 0.005 to 0.5 part by mass, and further more preferably 0.01 to 0.2 parts by mass with respect to 100 parts by mass of the ethylenically unsaturated monomer used for the polymerization. In a case where the addition amount of the flocculant is within the above-mentioned range, water-absorbent resin particles having a target particle size distribution can be easily obtained.

In the production of the water-absorbent resin particles, it is preferable to perform surface crosslinking of a surface portion (surface and in the vicinity of surface) of a hydrogel-like polymer using a surface crosslinking agent in a drying step (water removing step) or any subsequent steps. By performing surface crosslinking, it is easy to control the hardened bulk density, its amount of change, the water-absorbent characteristics (water retention amount and the like) and the like of the water-absorbent resin particles. The surface crosslinking is preferably performed at the timing when the hydrogel-like polymer has a specific water content. The timing of surface crosslinking is preferably when the water content of the hydrogel-like polymer is 5% to 50% by mass, more preferably when the water content of the hydrogel-like polymer is 10% to 40% by mass, and further more preferably when the water content of the hydrogel-like polymer is 15% to 35% by mass. The water content (mass%) of the hydrogel-like polymer is calculated by the following formula.

$$\text{Water content} = [\text{Ww}/(\text{Ww} + \text{Ws})] \times 100$$

Ww: water amount of a hydrogel-like polymer obtained by adding water amount used if necessary when mixing a flocculant, a surface crosslinking agent, or the like to an amount obtained by subtracting water amount discharged to the outside of the system in the drying step, from water amount contained in a monomer aqueous solution before polymerization in the entire polymerization step.

Ws: Solid content calculated from the charged amount of materials such as ethylenically unsaturated monomer, crosslinking agent, and initiator that constitute a hydrogel-like polymer.

Examples of the surface crosslinking agent include compounds having two or more reactive functional groups. Examples of the surface crosslinking agent include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylpropane triglycidyl ether (poly)propylene glycol polyglycidyl ether, and (poly)glycerol polyglycidyl ether; haloepoxy compounds such as epichlorohydrin, epibromhydrin, and α-methylepichlorohydrin; isocyanate compounds such as 2,4-tolylene diisocyanate and hexamethylenediisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis [N, N-di (β-hydroxyethyl)] adipamide. The surface crosslinking agent may be used alone, or may be used in combination of two or more. The surface crosslinking agent is preferably a polyglycidyl compound, and more preferably at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

The use amount of the surface crosslinking agent is preferably 0.01 to 20 mmol, more preferably 0.05 to 10 mmol, further more preferably 0.1 to 5 mmol, particularly preferably 0.15 to 1 mmol, and extremely preferably 0.2 to 0.5 mmol per 1 mol of the ethylenically unsaturated monomer used for polymerization, from a viewpoint of easily obtaining suitable hardened bulk density and its amount of change, water-absorbent characteristics (water retention amount and the like), and the like.

After surface crosslinking, it is possible to obtain polymer particles which are surface crosslinked dried products by distilling water and a hydrocarbon dispersion medium with a known method, heating and drying under reduced pressure, or the like.

As described above, the polymer particles contained in the water-absorbent resin particles can be obtained by using an internal crosslinking agent used at the time of polymerizing the monomer, and can be obtained by using an internal crosslinking agent, and an external crosslinking agent (a collective term for a post-polymerization crosslinking agent used after the polymerization of the monomer, and a surface crosslinking agent used in the drying step after polymerization of a monomer or subsequent steps) used after polymerization of the monomer. The ratio of the use amount of the external crosslinking agent with respect to the internal crosslinking agent (external crosslinking agent/internal crosslinking agent; crosslinking ratio) is preferably 5 to 100, more preferably 6 to 80, further more preferably 8 to 60, particularly preferably 10 to 40, extremely preferably 10 to 30, and extraordinarily preferably 10 to 25, from a viewpoint of easily obtaining suitable hardened bulk density and its amount of change, water-absorbent characteristics (water retention amount and the like), and the like. The water-absorbent resin particles may contain polymer particles which are reaction products using an internal crosslinking agent, and may contain polymer particles which are reaction products using an internal crosslinking agent and an external crosslinking agent. The ratio of the use amount of the external crosslinking agent to the internal crosslinking agent in the polymer particles is preferably in the above range.

In addition to the polymer particles, the water-absorbent resin particles of the present embodiment can further contain additional components such as a gel stabilizer, a metal chelating agent (ethylenediaminetetraacetic acid and a salt thereof, diethylenetriamine pentaacetate and a salt thereof, and the like, for example, diethylenetriamine pentasodium pentaacetate), and a flowability improver (lubricant). Additional components can be disposed inside the polymer particles, on the surface of the polymer particles, or both thereof.

The water-absorbent resin particles may contain a plurality of inorganic particles disposed on the surface of the polymer particles. For example, by mixing the polymer particles and the inorganic particles, it is possible to dispose the inorganic particles on the surface of the polymer particles. The inorganic particles may be silica particles such as amorphous silica.

In a case where the water-absorbent resin particles include inorganic particles disposed on the surface of the polymer particles, the content of the inorganic particles may be in the following range based on the total mass of the polymer particles. The content of the inorganic particles may be 0.05% by mass or more, or 0.1% by mass or more. The content of the inorganic particles may be 5.0% by mass or less, 3.0% by mass or less, 1.0% by mass or less, 0.5% by mass or less, 0.3% by mass or less, or 0.2% by mass or less.

The inorganic particles here usually have a minute size as compared with the size of the polymer particles. For example, the average particle diameter of the inorganic particles may be 0.1 to 50 μm, 0.5 to 30 μm, or 1 to 20 μm. The average particle diameter can be measured by a pore electric resistance method or a laser diffraction/scattering method depending on the characteristics of the particles.

The absorber of the present embodiment contains the water-absorbent resin particles of the present embodiment. The absorber of the present embodiment may contain a fibrous substance, for example, is a mixture containing water-absorbent resin particles and the fibrous substance. For example, the structure of the absorber may be a structure in which the water-absorbent resin particles and the fibrous substance are uniformly mixed, may be a structure in which the water-absorbent resin particles are sandwiched between the fibrous substances formed in the form of a sheet or a layer, or may be other structures.

Examples of the fibrous substance include finely pulverized wood pulp; cotton; cotton linter; rayon; cellulosic fibers such as cellulose acetate; synthetic fibers such as polyamide, polyester and polyolefin; and a mixture of these fibers. The fibrous substance may be used alone, or may be used in combination of two or more. As the fibrous substance, hydrophilic fibers can be used.

In order to enhance the morphological retention before and during use of the absorber, the fibers may be adhered to each other by adding an adhesive binder to the fibrous substance. Examples of the adhesive binder include thermal bonding synthetic fibers, hot melt adhesives, and adhesive emulsions. The adhesive binder may be used alone, or may be used in combination of two or more.

Examples of the thermal bonding synthetic fiber include a total fusion type binder such as polyethylene, polypropylene, and an ethylene-propylene copolymer; and a non-total fusion type binder made of a side-by-side or core-sheath structure of polypropylene and polyethylene. In the above-mentioned non-total fusion type binder, only the polyethylene portion can be thermal-bonded.

Examples of the hot melt adhesive include a mixture of a base polymer such as ethylene-vinyl acetate copolymer, styrene-isoprene-styrene block copolymer, styrene-butadiene-styrene block copolymer, styrene-ethylene-butylene-styrene block copolymer, styrene-ethylene-propylene-styrene block copolymer, and amorphous polypropylene with a tackifier, a plasticizer, an antioxidant, or the like.

Examples of the adhesive emulsion include a polymerization product of at least one monomer selected from the group consisting of methyl methacrylate, styrene, acrylonitrile, 2-ethylhexyl acrylate, butyl acrylate, butadiene, ethylene, and vinyl acetate.

The absorber of the present embodiment may contain an inorganic powder (for example, amorphous silica), a deodorant, an antibacterial agent, a dye, a pigment, a fragrance, a sticking agent, or the like. In a case where the water-absorbent resin particles contain inorganic particles, the absorber may contain an inorganic powder in addition to the inorganic particles of the water-absorbent resin particles.

The shape of the absorber of the present embodiment may be a sheet shape, for example. The thickness of the absorber (for example, thickness of the sheet shaped absorber) may be 0.1 to 20 mm or 0.3 to 15 mm.

The content of the water-absorbent resin particles in the absorber may be 2% to 100% by mass, 10% to 80% by mass, or 20% to 60% by mass with respect to the total of the water-absorbent resin particles and the fibrous substance, from a viewpoint of easily obtaining sufficient water absorption performance.

The content of the water-absorbent resin particles in the absorber is preferably 100 to 1000 g, more preferably 150 to 800 g, and further more preferably 200 to 700 g per 1 $m^2$ of the absorber from a viewpoint of easily obtaining sufficient water absorption performance. The content of the fibrous substance in the absorber is preferably 50 to 800 g, more preferably 100 to 600 g, and further more preferably 150 to 500 g per 1 $m^2$ of the absorber from a viewpoint of easily obtaining sufficient water absorption performance.

The water content of the absorber during the production of the absorbent article of the present embodiment is preferably in the following range. The water content is preferably 5% by mass or more, 10% by mass or more, 15% by mass or more, 16% by mass or more, 17% by mass or more, or 17.5% by mass or more, from a viewpoint of easily obtaining better moldability during the production of an absorbent article. The water content is preferably 20% by mass or less, 19% by mass or less, or 18% by mass or less from a viewpoint of easily obtaining better absorption performance when used in an absorbent article. From these viewpoints, the water content is preferably 5% to 20% by mass. The water content can be measured by the method described in examples to be described later.

The absorbent article of the present embodiment includes an absorber of the present embodiment. Examples of the absorbent article of the present embodiment include a core wrap that retains an absorber and prevents falloff or flow of a constituent member of the absorber; a liquid permeable sheet disposed on the outermost part at the side where the liquid to be absorbed enters; and a liquid impermeable sheet disposed on the outermost part at the opposite side to the side where the liquid to be absorbed enters. Examples of the absorbent article include diapers (for example, paper diapers), toilet training pants, incontinence pads, sanitary materials (sanitary napkins, tampons, and the like), sweat pads, pet sheets, portal toilet members, and animal excrement treatment materials.

Fig. 1 is a cross-sectional view showing an example of an absorbent article. An absorbent article 100 shown in Fig. 1 includes an absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown so that there is a gap between the members, but the members may be in close contact with each other without the gap.

The absorber 10 has a water-absorbent resin particle 10a of the present embodiment and a fiber layer 10b containing a fibrous substance. The water-absorbent resin particles 10a are dispersed in the fiber layer 10b.

The core wrap 20a is disposed on one surface side of the absorber 10 (upper side of the absorber 10 in Fig. 1) in a state of being in contact with the absorber 10. The core wrap 20b is disposed on the other surface side of the absorber 10 (lower side of the absorber 10 in Fig. 1) in a state of being in contact with the absorber 10. The absorber 10 is disposed between the core wrap 20a and the core wrap 20b. Examples of the core wraps 20a and 20b include tissues, non-woven fabrics, woven fabrics, synthetic resin films having liquid permeation holes, and net-like sheets having a mesh. The core wrap 20a and the core wrap 20b have a main surface having the same size as that of the absorber 10, for example.

The liquid permeable sheet 30 is disposed on the outermost part at the side where the liquid to be absorbed enters. The liquid permeable sheet 30 is disposed on the core wrap 20a in a state of being in contact with the core wrap 20a. Examples of the liquid permeable sheet 30 include a non-woven fabric made of a synthetic resin such as polyethylene, polypropylene, polyester, and polyamide, and a porous sheet. The liquid impermeable sheet 40 is disposed on the outermost part at the opposite side to the liquid permeable sheet 30 in the absorbent article 100. The liquid impermeable sheet 40 is disposed on a lower side of the core wrap 20b in a state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. The liquid permeable sheet 30 and the liquid impermeable sheet 40 have a main surface wider than the main surface of the absorber 10, and outer edges of the liquid permeable sheet 30 and the liquid impermeable sheet 40 are present around the absorber 10 and the core wraps 20a and 20b.

The magnitude relationship between the absorber 10, the core wraps 20a and 20b, the liquid permeable sheet 30, and the liquid impermeable sheet 40 is not particularly limited, and is appropriately adjusted according to the use of the absorbent article or the like. In addition, the method of retaining the shape of the absorber 10 using the core wraps 20a and 20b is not particularly limited, and as shown in Fig. 1, the absorber may be wrapped by a plurality of core wraps, and the absorber is wrapped by one core wrap.

The absorber may be adhered to a top sheet. In a case where the absorber is sandwiched or covered by the core wrap, it is preferable that at least the core wrap and the top sheet are adhered to each other, and it is more preferable that the core wrap and the top sheet are adhered to each other and the core wrap and the absorber are adhered to each other. Examples of a method of adhering the absorber include a method of adhering by applying a hot melt adhesive to the top sheet at predetermined intervals in a striped shape, a spiral shape, or the like in a width direction; and a method of adhering using a water-soluble binder such as starch, carboxymethyl cellulose, polyvinyl alcohol, polyvinylpyrrolidone, and other water-soluble polymers. In addition, in a case where the absorber contains thermal bonding synthetic fibers, a method of adhering by thermal bonding of the thermal bonding synthetic fibers may be adopted.

According to the present embodiment, it is possible to provide a liquid absorbing method using the water-absorbent resin particles, the absorber or the absorbent article of the present embodiment. The liquid absorbing method of the present embodiment includes a step of bringing the liquid to be absorbed into contact with the water-absorbent resin particles, the absorber or the absorbent article of the present embodiment.

According to the present embodiment, it is possible to provide a method for improving the permeation rate of an absorbent article, which is a method for improving the permeation rate using the water-absorbent resin particles, the absorber or the absorbent article of the present embodiment. According to the present embodiment, it is possible to provide a method for producing water-absorbent resin particles, including a selection step of selecting water-absorbent resin particles based on the hardened bulk density and its amount of change (amount of change of hardened bulk density when the water-absorbent resin particles are caused to absorb moisture at the temperature of 30°C and the relative humidity of 80% for 1 hour). In the selection step, for example, the water-absorbent resin particles are selected based on whether or not the hardened bulk density is 0.500 g/cm$^3$ or more, and the amount of change in the hardened bulk density at the time of moisture absorption at the temperature of 30°C and the relative humidity of 80% for 1 hour is 0.020 g/cm$^3$ or more.

**Examples**

Hereinafter, contents of the present invention will be described in further detail using examples and comparative examples, but the present invention is not limited to the following examples.

<Preparation of water-absorbent resin particles>

(Example 1)

A round-bottomed cylindrical separable flask with the inner diameter of 11 cm and the internal volume of 2 L

equipped with a reflux cooling device, a dropping funnel, a nitrogen gas introduction tube, and a stirrer (a stirrer blade having two stages of four inclined paddle blades with the blade diameter of 5 cm) was prepared. Into this flask, 293 g of n-heptane was added as a hydrocarbon dispersion medium and 0.736 g of a maleic anhydride-modified ethylene/propylene copolymer (manufactured by Mitsui Chemicals, Inc., High Wax 1105A) was added as a polymeric dispersant to obtain a mixture. The dispersant was dissolved by raising the temperature to 80°C while stirring the mixture, and then the mixture was cooled to 50°C.

Subsequently, 92.0 g of 80.5% by mass aqueous acrylic acid solution (acrylic acid: 1.03 mol) was added into a beaker having the internal volume of 300 mL as a water-soluble ethylenically unsaturated monomer. Subsequently, while cooling from the outside, 147.7 g of 20.9% by mass sodium hydroxide aqueous solution was added dropwise into the beaker to perform 75 mol% of neutralization on acrylic acid. Thereafter, 0.092 g of hydroxyethyl cellulose (manufactured by Sumitomo Seika Chemicals Co., Ltd., HEC AW-15F) as a thickener, 0.092 g (0.339 mmol) of 2,2'-azobis (2-amidinopropane) dihydrochloride and 0.018 g (0.068 mmol) of potassium persulfate as a water-soluble radical polymerization initiator, and 0.005 g (0.029 mmol) of ethylene glycol diglycidyl ether as an internal crosslinking agent were added and then dissolved therein to prepare a first stage aqueous solution.

Then, the first stage aqueous solution was added into the above-mentioned separable flask while stirring at the rotation speed of 550 rpm of the stirrer, and then stirring was performed for 10 minutes. Thereafter, a surfactant solution obtained by heat-dissolving 0.736 g of sucrose stearic acid ester (surfactant, manufactured by Mitsubishi-Chemical Foods Corporation, Ryoto Sugar Ester S-370, HLB value: 3) in 6.62 g of n-heptane was added into the separable flask. Then, the inside of the system was sufficiently replaced with nitrogen while stirring at the stirring speed of 550 rpm of the stirrer. Thereafter, the flask was immersed in a water bath at 70°C to raise the temperature, and polymerization was performed for 60 minutes to obtain a first stage polymerization slurry solution.

Subsequently, 128.8 g of 80.5% by mass aqueous acrylic acid solution (acrylic acid: 1.43 mol) was added into another beaker having the internal volume of 500 mL as a water-soluble ethylenically unsaturated monomer. Subsequently, while cooling from the outside, 159.0 g of 27% by mass sodium hydroxide aqueous solution was added dropwise into the beaker to perform 75 mol% of neutralization on acrylic acid. Thereafter, 0.129 g (0.475 mmol) of 2,2'-azobis (2-amidinopropane) dihydrochloride and 0.026 g (0.095 mmol) of potassium persulfate as water-soluble radical polymerization initiators were added and then dissolved therein to prepare a second stage aqueous solution.

Subsequently, while stirring at the rotation speed of 1000 rpm of the stirrer, the inside of the above-mentioned separable flask was cooled to 25°C, and then the total amount of the above-mentioned second stage aqueous solution was added to the above-mentioned first stage polymerization slurry solution. Subsequently, after replacing the inside of the system with nitrogen for 30 minutes, the flask was immersed in a water bath at 70°C again to raise the temperature, and the polymerization reaction was performed for 60 minutes. Thereafter, 0.580 g of 2% by mass ethylene glycol diglycidyl ether aqueous solution (ethylene glycol diglycidyl ether: 0.067 mmol) was added as a post-polymerization crosslinking agent to obtain a second stage hydrogel-like polymer.

To the above-mentioned second stage hydrogel-like polymer, 0.265 g of 45% by mass diethylenetriamine pentasodium pentaacetate aqueous solution was added under stirring. Thereafter, the temperature of the reaction solution was raised in an oil bath at 125°C, and 217.8 g of water was extracted to the outside of the system while refluxing n-heptane by azeotropic distillation of n-heptane and water. Then, 4.42 g of 2% by mass ethylene glycol diglycidyl ether aqueous solution (ethylene glycol diglycidyl ether: 0.507 mmol) was added into the flask as a surface crosslinking agent, and then the mixture was held at 83°C for 2 hours.

Thereafter, n-heptane was evaporated at 125°C and dried to obtain polymer particles (dried product). After passing the polymer particles through a sieve having the opening of 850 μm, 0.2% by mass of amorphous silica (Tokusil NP-S manufactured by Oriental Silicas Corporation) was mixed with the polymer particles based on the total mass of the polymer particles to obtain 229.6 g of water-absorbent resin particles. The medium particle diameter of the water-absorbent resin particles was 342 μm. The ratio of the use amount of the external crosslinking agent to the use amount of the internal crosslinking agent was 19.8 in terms of molar ratio.

(Example 2)

230.8 g of water-absorbent resin particles were obtained in the same manner as that in Example 1, except that, in the preparation of the first stage aqueous solution, without using 2,2'-azobis (2-amidinopropane) dihydrochloride, 0.0736 g (0.272 mmol) of potassium persulfate was used as a water-soluble radical polymerization initiator, and 0.010 g (0.057 mmol) of ethylene glycol diglycidyl ether was used as an internal crosslinking agent; in the preparation of the first stage polymerization slurry solution, the rotation speed of the stirrer was changed to 500 rpm; in the preparation of the second stage aqueous solution, without using 2,2'-azobis (2-amidinopropane) dihydrochloride, 0.090 g (0.334 mmol) of potassium persulfate was used as a water-soluble radical polymerization initiator; in the hydrogel-like polymer after the second stage polymerization, 256.1 g of water was extracted to the outside of the system by azeotropic distillation; and 0.1% by mass amorphous silica with respect to the mass of the polymer particles was mixed with the polymer particles. The medium particle diameter of the water-absorbent resin particles was 349 $\mu$m. The ratio of the use amount of the external crosslinking agent with respect to the use amount of the internal crosslinking agent was 10.1 in terms of molar ratio.

(Example 3)

228.8 g of water-absorbent resin particles were obtained in the same manner as that in Example 2, except that, in the preparation of the hydrogel-like polymer, the temperature in the separable flask was changed from 25°C to 28°C, and in the hydrogel-like polymer after the second stage polymerization, 256.8 g of water was extracted to the outside of the system by azeotropic distillation. The medium particle diameter of the water-absorbent resin particles was 321 $\mu$m.

(Example 4)

A round-bottomed cylindrical separable flask with the inner diameter of 11 cm and the internal volume of 2 L equipped with a reflux cooling device, a dropping funnel, a nitrogen gas introduction tube, and a stirrer (a stirrer blade having two stages of four inclined paddle blades with the blade diameter of 5 cm) was prepared. To this flask, 281 g of cyclohexane as a hydrocarbon dispersion medium was added, and 0.564 g of ethyl cellulose (Aqualon N100, manufactured by Ashland) was added to obtain a mixture. While stirring this mixture at the rotation speed of 700 rpm of the stirrer, nitrogen gas was blown into the system to expel dissolved oxygen, and then the flask was immersed in a water bath at 80°C to raise the temperature to 75°C.

Subsequently, 92.0 g (1.28 mol) of 100% acrylic acid was added into a beaker having the internal volume of 300 mL as a water-soluble ethylenically unsaturated monomer. Subsequently, while cooling from the outside, 142.0 g of 27% by mass sodium hydroxide aqueous solution was added dropwise into the beaker to perform 75 mol% of neutralization on acrylic acid. Thereafter, a solution obtained by dissolving 0.184 g (0.681 mmol) of potassium persulfate as a radical polymerization initiator in 14.8 g of water was added. Then, a monomer aqueous solution was prepared by blowing nitrogen gas and removing oxygen remaining in the aqueous solution.

Then, using a tube pump, the above-mentioned monomer aqueous solution was added dropwise into the above-mentioned separable flask over 1 hour to perform a polymerization reaction. Thereafter, the flask was immersed in an oil bath set at 125°C while stirring at the rotation speed of 1000 rpm of the stirrer, and 95.8 g of water was extracted to the outside of the system while refluxing cyclohexane by azeotropic distillation of cyclohexane and water.

Thereafter, in the above-mentioned separable flask, 0.036 g (0.176 mmol) of polyglycerol polyglycidyl ether (Denacol EX-512 manufactured by Nagase Chemtex Corporation) was added together with 3.61 g of water as a surface crosslinking agent and dissolved therein. Then, the reaction was performed at 75°C to 80°C for 1 hour while heating in an oil bath. Immediately after the reaction, the cyclohexane phase was filtered off with a sieve having the opening of 38 $\mu$m to obtain a water-absorbent resin hydrate.

This water-absorbent resin hydrate was heat-dried under pressure at 0.006 MPa with a decompression drier set at 90°C to obtain polymer particles (dried product). After passing these polymer particles through a sieve having the opening of 850 $\mu$m, 0.5% by mass of amorphous silica (Tokusil NP-S manufactured by Oriental Silicas Corporation) was mixed with the polymer particles based on the total mass of the polymer particles to obtain 104.0 g of water-absorbent resin particles. The medium particle diameter of the water-absorbent resin particles was 370 $\mu$m. Since no internal crosslinking agent was used, the crosslinking ratio could not be calculated.

(Comparative Example 1)

229.0 g of water-absorbent resin particles were obtained in the same manner as that in Example 2, except that, in the preparation of the first stage polymerization slurry solution, the rotation speed of the stirrer was changed to 550 rpm; in the preparation of the second stage aqueous solution, in addition to adding 0.090 g (0.334 mmol) of potassium persulfate as a water-soluble radical polymerization initiator, 0.0116 g (0.067 mmol) of ethylene glycol diglycidyl ether was added as an internal crosslinking agent; in the preparation of the hydrogel-like polymer, the temperature in the separable flask was changed from 25°C to 31°C, and 0.265 g of 45% by mass diethylenetriamine pentasodium pentaacetate aqueous solution was added after performing the polymerization reaction for 60 minutes, without adding the crosslinking agent; and in the hydrogel-like polymer after the second stage polymerization, 256.5 g of water was extracted to the outside of the system by azeotropic distillation. The medium particle diameter of the water-absorbent resin particles was 146 $\mu$m. The ratio of the use amount of the external crosslinking agent with respect to the use amount of the internal crosslinking agent was 4.1 in terms of molar ratio.

(Comparative Example 2)

A round-bottomed cylindrical separable flask having 4 side wall baffles (baffle width: 7 mm), with the inner diameter of 11 cm and the internal volume of 2 L, equipped with a reflux cooling device, a dropping funnel, a nitrogen gas introduction tube, and a stirrer (a stirrer blade having two stages of four inclined paddle blades with the blade diameter of 5 cm (those surface-treated with fluororesin)) was prepared. Into this flask, 451.4 g of n-heptane as a hydrocarbon dispersion medium was added, and 1.288 g of sorbitan monolaurate (Nonion LP-20R, HLB value: 8.6, manufactured by NOF CORPORATION) was added as a surfactant to obtain a mixture. The sorbitan monolaurate was dissolved in n-heptane by raising the temperature to 50°C while stirring this mixture at the rotation speed of 300 rpm of the stirrer, and then the mixture was cooled to 40°C.

Subsequently, 92.0 g of 80.5% by mass aqueous acrylic acid solution (acrylic acid: 1.03 mol) was put into a triangular flask having the internal volume of 500 mL. Subsequently, while cooling with ice from the outside, 147.7 g of 20.9% by mass sodium hydroxide aqueous solution was added dropwise to neutralize the acrylic acid for obtaining an acrylic acid partial neutralization aqueous solution. Subsequently, 0.1012 g (0.374 mmol) of potassium persulfate as a water-soluble radical polymerization initiator was added to the acrylic acid partial neutralization aqueous solution, and then dissolved therein to prepare a monomer aqueous solution.

After adding the above-mentioned monomer aqueous solution into the above-mentioned separable flask, the inside of the system was sufficiently replaced with nitrogen. Thereafter, while stirring at the rotation speed of 700 rpm of the stirrer, the flask was immersed in a water bath at 70°C and held for 60 minutes to complete the polymerization to obtain a hydrogel-like polymer.

Thereafter, while stirring at the stirring speed of 1000 rpm of the stirrer, a dispersion obtained by previously dispersing 0.092 g of amorphous silica (Oriental Silicas Corporation, Tokusil NP-S) as a powdered inorganic flocculant to 100 g of n-heptane was added to a polymer solution containing the produced hydrogel-like polymer, n-heptane, and a surfactant, and then mixing was performed for 10 minutes. Thereafter, the flask containing the reaction solution was immersed in an oil bath at 125°C, and 129.0 g of water was extracted to the outside of the system while refluxing n-heptane by azeotropic distillation of n-heptane and water. Thereafter, 4.14 g of 2% by mass ethylene glycol diglycidyl ether aqueous solution (ethylene glycol diglycidyl ether: 0.475 mmol) was added as a surface crosslinking agent, and then the internal temperature was held at 83 ± 2°C for 2 hours.

Thereafter, water and n-heptane were evaporated at 120°C and dried until almost no evaporated product from the system was distilled off to obtain polymer particles (dried product). These polymer particles were passed through a sieve having the opening of 850 $\mu$m to obtain 90.1 g of water-absorbent resin particles. The medium particle diameter of the water-absorbent resin particles was 360 $\mu$m. Since no internal crosslinking agent was used, the crosslinking ratio could not be calculated.

(Comparative Example 3)

After passing the polymer particles of Comparative Example 2 through a sieve having the opening of 850 $\mu$m, 2.0 % by mass of amorphous silica (Tokusil NP-S manufactured by Oriental Silicas Corporation) was mixed with the polymer particles based on the total mass of the polymer particles to obtain 91.9 g of water-absorbent resin particles. The medium particle diameter of the water-absorbent resin particles was 357 $\mu$m. Since no internal crosslinking agent was used, the crosslinking ratio could not be calculated.

<Measurement of medium particle diameter>

The above-mentioned medium particle diameter of the water-absorbent resin particles was measured by the following procedure. Specifically, JIS standard sieves were combined in the following order from the top: a sieve having the opening of 600 $\mu$m, a sieve having the opening of 500 $\mu$m, a sieve having the opening of 425 $\mu$m, a sieve having the opening of 300 $\mu$m, a sieve having the opening of 250 $\mu$m, a sieve having the opening of 180 $\mu$m, a sieve having the opening of 150 $\mu$m, and a tray. 50 g of the water-absorbent resin particles were put in the topmost sieve among the combined sieves, and classification was performed by shaking for 10 minutes using a Ro-tap shaker. After classification, the mass of the particles remaining on each sieve was calculated as a mass percentage with respect to the total amount, and the particle size distribution was obtained. The relationship between the opening of the sieve and the integrated value of the mass percentage of the particles remaining on the sieve was plotted on logarithmic probability paper by integrating in the order from the one having the largest particle diameter on the sieve with respect to this particle size distribution. By connecting the plots on the probability paper with a straight line, the particle diameter corresponding to the cumulative mass percentage of 50% by mass was obtained as the medium particle diameter.

<Hardened bulk density of water-absorbent resin particles>

Using a powder property evaluation device (model number: PT-X, manufactured by Hosokawa Micron Co., Ltd.), the hardened bulk density of the water-absorbent resin particles before (initial state) and after moisture absorption was measured by the following procedure. The measurement of the hardened bulk density was performed under a condition of room temperature (25°C ± 2°C).

First, a mass W0 of the empty container (cap XS-18, inner diameter 5.0 cm, height 5.0 cm, volume 100 cm³) was measured. Subsequently, after attaching the cap XS-17 (cylindrical shape having the inner diameter of 5.1 cm and the height of 5.1 cm) to the container, 100 g of the water-absorbent resin particles were added using the scoop XS-12 attached to the device. Subsequently, after disposing on a tapping lift bar attached to the device, an impact was applied by performing 180 times of tapping under a condition of the stroke of 18 mm. Then, after removing the cap, the water-absorbent resin particles raised from the container were scraped off and removed using a blade XS-13. Subsequently, a mass W1 of the container containing the water-absorbent resin particles was measured. Based on the mass W0 and the mass W1, the hardened bulk density was obtained by the following formula. The hardened bulk density was measured three times in total, and the average value was obtained as the hardened bulk density SO [g/cm³] of the water-absorbent resin particles before moisture absorption.

$$\text{Hardened bulk density [g/cm}^3\text{]} = (\text{W1 [g]} - \text{W0 [g]})/100\ [\text{cm}^3]$$

In addition, 100 g of the water-absorbent resin particles were uniformly scattered on a bat having a smooth bottom surface (bottom surface size: 29.5 cm × 23.0 cm, height: 4.5 cm, made of SUS) to obtain a sample. Subsequently, the above-mentioned sample was left for 1 hour and caused to absorb moisture in a constant temperature constant humidity chamber (model number: LH21-11M, manufactured by Nagano Science Co., Ltd.) adjusted to conditions of 30 ± 1°C and the relative humidity of 80 ± 5%. The water-absorbent resin particles after moisture absorption were collected, and the hardened bulk density S1 [g/cm³] of the water-absorbent resin particles after moisture absorption was measured by the same procedure as the above-mentioned procedure.

Then, by subtracting the hardened bulk density SO from the hardened bulk density S1, an amount of change in the hardened bulk density [g/cm³] after moisture absorption for 1 hour was obtained. The results are shown in Table 1.

<Water content of water-absorbent resin particles>

The water content of the water-absorbent resin particles before and after moisture absorption was measured by the following procedure.

First, the mass of the container (aluminum wheel case, No. 8) was measured. Subsequently, after housing the water-absorbent resin particles (about 2 g) in the container, a total amount of the container and the water-absorbent resin particles was measured. The mass Wa [g] of the water-absorbent resin particles was obtained by subtracting the mass of the container from the total amount.

Subsequently, the container housing the water-absorbent resin particles was dried for 2 hours in a hot air drier (model number: DRE320DB, manufactured by ADVANTEC) set at the internal temperature of 105°C, and then allowed to cool in a desiccator. Subsequently, the total amount of the container and the water-absorbent resin particles was measured, and the mass of the container was subtracted from the total amount to obtain a mass Wb [g] of the water-absorbent resin particles after drying.

Then, a water content C1 of the water-absorbent resin particles before moisture absorption was calculated by the following formula. The results are shown in Table 1.

$$\text{Water content before moisture absorption C1 [mass\%]} = [(\text{Wa}-\text{Wb})/\text{Wa}] \times 100$$

In addition, the water-absorbent resin particles were caused to absorb moisture in the same procedure as the above-mentioned procedure of the hardened bulk density of the water-absorbent resin particles, and then a mass Wc of the water-absorbent resin particles was measured. By subtracting the mass Wa from the mass Wc, a mass increase ΔW of the water-absorbent resin particles before and after the moisture absorption test was calculated. Then, a water content of the water-absorbent resin particles after moisture absorption C2 was calculated by the following formula. The results are shown in Table 1.

$$\text{Water content after moisture absorption C2 [mass\%]} = [\{(\text{water content before moisture absorption C1 [mass\%]} \times \text{mass of water-absorbent resin particles Wa [g]}) + \text{mass increase of water-absorbent resin particles before and after the moisture absorption test } \Delta W \text{ [g]}\}/\text{mass of water-absorbent resin particles after moisture absorption Wc [g]]} \times 100$$

<Water retention amount of water-absorbent resin particles>

The water retention amount (room temperature, 25 ± 2°C) of physiological saline of the water-absorbent resin particles was measured by the following procedure. First, a cotton bag (Membroroad No. 60, width 100 mm × length 200 mm) containing 2.0 g of the weighted water-absorbent resin particles was placed in a beaker having the internal volume of 500 mL. After pouring 500 g of 0.9% by mass sodium chloride aqueous solution (physiological saline) into a cotton bag containing the water-absorbent resin particles at one time so that lumps cannot be formed, an upper portion of the cotton bag was tied with a rubber band and the cotton bag was left for 30 minutes to swell the water-absorbent resin particles. The cotton bag after 30 minutes was dehydrated for 1 minute using a dehydrator (product number: H-122, manufactured by KOKUSAN Co., Ltd.) set to have the centrifugal force of 167 G, and then the mass Wd [g] of the cotton bag containing swollen gel after dehydration was measured. The same operation was performed without adding the water-absorbent resin particles, the empty mass We [g] at the time when the cotton bag was wet was measured, and the water retention amount of physiological saline of the water-absorbent resin particles was calculated from the formula below. The results are shown in Table 1.

$$\text{Water retention amount [g/g]} = (\text{Wd - We})/2.0$$

<Evaluation of absorber performance>

(Preparation of test solution)

60 g of sodium chloride, 1.8 g of calcium chloride dihydrate, 3.6 g of magnesium chloride hexahydrate, and an appropriate amount of distilled water were put in a container having the internal volume of 10 L, and then completely dissolved. Subsequently, after adding 0.02 g of polyoxyethylene nonylphenyl ether, a total mass of the aqueous solution was adjusted to 6000 g by adding distilled water. Subsequently, a test solution (artificial urine) was obtained by coloring with a small amount of blue No. 1.

(Preparation of absorber)

Using an air flow type mixer (Padformer, manufactured by O-tec Co., Ltd.), 10 g of water-absorbent resin particles and 10 g of pulverized pulp were uniformly mixed by air papermaking to prepare a sheet-shaped absorber having the size of 40 cm × 12 cm.

(Water content of pulverized pulp)

A water content C3 of the pulverized pulp before moisture absorption was measured by the same procedure as the procedure for measuring the water content C1 of the water-absorbent resin particles before moisture absorption. The water content C3 was 6.3% by mass.

(Preparation of absorbent article)

After the absorber was laminated on the first tissue paper having the basis weight of 16 g/m$^2$ and having the same size as the absorber to obtain the first laminate, a moisture absorption operation was performed according to the method to be described later. Subsequently, a second tissue paper having the basis weight of 16 g/m$^2$ and having the

same size as the absorber was disposed on the absorber in the first laminate after moisture absorption. A second laminate was obtained by applying the load of 196 kPa to the entire body for 30 seconds in a state where the upper and lower sides of the absorber were sandwiched between tissue papers. In addition, an air-through type porous liquid permeable sheet made of polyethylene-polypropylene having the same size as that of the absorber and having the basis weight of 22 g/m$^2$ was disposed on the upper surface of the second laminate to prepare an absorbent article.

(Measurement of moisture absorption and water content of absorber)

The above-mentioned first laminate containing the absorber was placed on a 40 cm $\times$ 12 cm mat board (cardboard). Subsequently, after putting the first laminate together with the mat board in a constant temperature constant humidity chamber (model number: LH21-11M, manufactured by Nagano Science Co., Ltd.) under the conditions of 30 $\pm$ 1°C and the relative humidity of 80 $\pm$ 5%, moisture absorption was performed for 1 hour. Based on the mass of the first laminate before and after moisture absorption, the water content [mass%] of the absorber was calculated by the following formula. The results are shown in Table 1.

$$\text{Water content of absorber [mass\%]} = [\{(\text{water content of water-absorbent resin particles before moisture absorption C1 [mass\%]} \times \text{mass [g] of water-absorbent resin particles}) + (\text{water content of pulverized pulp before moisture absorption C3 [mass\%]} \times \text{mass of pulverized pulp [g]]} + \text{mass increase of absorber before and after moisture absorption test [g]}\}/\text{mass of absorber after moisture absorption [g]]} \times 100$$

(Evaluation of permeation rate)

In a room having the temperature of 25 $\pm$ 2°C, a measurement instrument equipped with a liquid injection cylinder having the inner diameter of 3 cm was placed in a central portion of an absorbent article disposed on a horizontal table. Subsequently, 80 mL of the test solution adjusted to 25 $\pm$ 1°C was injected into the cylinder at one time (supplied from a vertical direction) and the stopwatch was started. An absorption time from the start of injection until the test solution was completely absorbed in the absorber was measured. This operation was performed twice more at intervals of 30 minutes (three times in total), and the total value of the absorption time was obtained as the permeation rate (unit: second). The shorter the permeation rate, the better it is. The results are shown in Table 1.

[Table 1]

| | Water-absorbent resin particles | | | | | | | Absorber | Absorbent article |
| | Hardened bulk density [g/cm$^3$] | | | Water content [mass%] | | Water retention amount [g/g] | Medium particle diameter [μm] | Water content [mass%] | |
| | Before moisture absorption | After moisture absorption | Amount of change | Before moisture absorption | After moisture absorption | | | After moisture absorption | Permeation rate [sec] |
|---|---|---|---|---|---|---|---|---|---|
| Example 1 | 0.874 | 0.834 | 0.040 | 9 | 13 | 44 | 342 | 17.7 | 72 |
| Example 2 | 0.835 | 0.793 | 0.042 | 9 | 14 | 41 | 349 | 18.3 | 81 |
| Example 3 | 0.846 | 0.811 | 0.035 | 8 | 14 | 40 | 321 | 17.6 | 70 |
| Example 4 | 0.969 | 0.921 | 0.049 | 8 | 14 | 39 | 370 | 17.2 | 94 |
| Comparative Example 1 | 0.970 | 0.952 | 0.018 | 4 | 11 | 54 | 146 | 15.0 | 123 |
| Comparative Example 2 | 0.464 | 0.448 | 0.016 | 5 | 13 | 37 | 360 | 17.0 | 104 |
| Comparative Example 3 | 0.468 | 0.438 | 0.031 | 5 | 11 | 37 | 357 | 17.8 | 109 |

According to Table 1, it is confirmed that the water-absorbent resin particles having an appropriate hardened bulk density and its amount of change at the time of moisture absorption are effective in obtaining an absorbent article having a better permeation rate.

**Reference Signs List**

10: absorber, 10a: water-absorbent resin particle, 10b: fiber layer, 20a, 20b: core wrap, 30: liquid permeable sheet, 40: liquid impermeable sheet, 100: absorbent article.

**Claims**

1.  Water-absorbent resin particles comprising:

     polymer particles comprising a crosslinking polymer comprising a structural unit derived from an ethylenically unsaturated monomer; and
     inorganic particles disposed on a surface of the polymer particles,
     wherein the ethylenically unsaturated monomer comprises at least one compound selected from the group consisting of (meth)acrylic acid and a salt thereof,
     a ratio of the (meth)acrylic acid and the salt thereof is 70 to 100 mol% with respect to a total amount of monomers for obtaining the water-absorbent resin particles,
     a hardened bulk density is 0.500 $g/cm^3$ or more, and
     an amount of change in the hardened bulk density at a time of moisture absorption at a temperature of 30°C and a relative humidity of 80% for 1 hour is 0.020 $g/cm^3$ or more.

2.  The water-absorbent resin particles according to claim 1,
    wherein the hardened bulk density is 0.500 to 0.950 $g/cm^3$.

3.  The water-absorbent resin particles according to claim 1,
    wherein the hardened bulk density is 0.830 to 0.950 $g/cm^3$.

4.  The water-absorbent resin particles according to any one of claims 1 to 3,
    wherein a water retention amount of physiological saline is 30 to 55 g/g.

5.  The water-absorbent resin particles according to any one of claims 1 to 4,
    wherein a medium particle diameter is 250 to 600 $\mu$m.

6.  An absorber comprising:
    the water-absorbent resin particles according to any one of claims 1 to 5.

7.  An absorbent article comprising:
    the absorber according to claim 6.

8.  The absorbent article according to claim 7, which is a diaper.

Fig.1

## INTERNATIONAL SEARCH REPORT

| International application No. |
|---|
| PCT/JP2019/048804 |

**A. CLASSIFICATION OF SUBJECT MATTER**

C08F 20/06(2006.01)i; C08J 3/12(2006.01)i; A61F 13/53(2006.01)i; B01J 20/26(2006.01)i; B01J 20/28(2006.01)i
FI: C08J3/12 Z GEY; C08F20/06; A61F13/53 300; B01J20/26 D; B01J20/28 Z

According to International Patent Classification (IPC) or to both national classification and IPC

**B. FIELDS SEARCHED**

Minimum documentation searched (classification system followed by classification symbols)

C08J3/00-3/28; C08F6/00-246/00,301/00; A61L15/16-15/64; A61F13/15-13/84; B01J20/26

Documentation searched other than minimum documentation to the extent that such documents are included in the fields searched

Published examined utility model applications of Japan 1922–1996
Published unexamined utility model applications of Japan 1971–2020
Registered utility model specifications of Japan 1996–2020
Published registered utility model applications of Japan 1994–2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2005/075070 A1 (NIPPON SHOKUBAI CO., LTD.) 18.08.2005 (2005-08-18) entire text | 1-8 |
| A | JP 2003-88552 A (SUMITOMO SEIKA CHEMICALS CO., LTD.) 25.03.2003 (2003-03-25) entire text | 1-8 |
| A | WO 2018/181565 A1 (SUMITOMO SEIKA CHEMICALS CO., LTD.) 04.10.2018 (2018-10-04) entire text | 1-8 |

☐ Further documents are listed in the continuation of Box C.   ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 02 March 2020 (02.03.2020) | 17 March 2020 (17.03.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

<table>
<tr><td colspan="2"><b>INTERNATIONAL SEARCH REPORT</b><br>Information on patent family members</td><td>International application No.<br><br>PCT/JP2019/048804</td></tr>
</table>

| Patent Documents referred in the Report | Publication Date | Patent Family | Publication Date |
|---|---|---|---|
| WO 2005/075070 A1 | 18 Aug. 2005 | US 2005/0209352 A1<br>EP 1721663 A1<br>AU 2005210411 A<br>MX PA06008956 A<br>KR 10-2007-0004669 A<br>CN 1917954 A | |
| JP 2003-88552 A | 25 Mar. 2003 | (Family: none) | |
| WO 2018/181565 A1 | 04 Oct. 2018 | CN 110446726 A<br>KR 10-2019-0127698 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H6345819 A
- JP H9510889 A